(19) Europäisches Patentamt
European Patent Office
Office européen des brevets

(11) **EP 3 747 533 A1**

(12) **EUROPEAN PATENT APPLICATION**
published in accordance with Art. 153(4) EPC

(43) Date of publication:
**09.12.2020 Bulletin 2020/50**

(51) Int Cl.:
**B01D 53/50** (2006.01)　　**B01D 53/14** (2006.01)
**B01D 53/78** (2006.01)

(21) Application number: **19764975.9**

(22) Date of filing: **28.02.2019**

(86) International application number:
**PCT/JP2019/007893**

(87) International publication number:
**WO 2019/172088 (12.09.2019 Gazette 2019/37)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **06.03.2018　JP 2018039287**

(71) Applicant: **Mitsubishi Power, Ltd.
Yokohama-shi, Kanagawa 220-8401 (JP)**

(72) Inventors:
• **KAMIYAMA, Naoyuki
Tokyo 108-8215 (JP)**
• **KAGAWA, Seiji
Tokyo 108-8215 (JP)**
• **USHIKU, Tetsu
Yokohama-shi, Kanagawa 220-8401 (JP)**
• **MORI, Shogo
Yokohama-shi, Kanagawa 220-8401 (JP)**

(74) Representative: **Henkel & Partner mbB
Patentanwaltskanzlei, Rechtsanwaltskanzlei
Maximiliansplatz 21
80333 München (DE)**

(54) **OPERATION SUPPORT SYSTEM AND OPERATION SUPPORT METHOD FOR DESULFURIZATION EQUIPMENT**

(57)　　Provided is an operation support system for a desulfurization apparatus evaluates soundness of the desulfurization apparatus by comparing an analytic performance calculated based on operation data of the desulfurization apparatus and a measured performance and creates support information including an operation condition candidate set based on the evaluation result and a balance forecast.

FIG. 4

**Description**

TECHNICAL FIELD

This disclosure relates to an operation support system and an operation support method for a desulfurization apparatus to perform a desulfurization process on exhaust gas using an absorbent.

BACKGROUND

Some plant facilities involving combustion of fuel are equipped with a desulfurization apparatus to remove $SO_2$ (sulfur dioxide) contained in exhaust gas. In a desulfurization apparatus employing a wet lime-gypsum method, which is one of the methods, $SO_2$ contained in the exhaust gas is removed by bringing absorption liquid, in which limestone powder is slurried in a suspended state as an absorbent, into contact with the exhaust gas while circulating the absorption liquid with a circulation pump

In such a desulfurization apparatus, trial operations are performed under various assumed operation conditions, and the operation condition under which maximum performance can be exhibited is grasped based on date collected during the trial operations, which is reflected in actual plant operations. Further, when evaluating the operation conditions of the desulfurization apparatus, it is important to maximize an economic benefit of a user by considering balance including utility power costs involved in the operation of the desulfurization apparatus and sale profits to third parties of byproducts such as gypsum generated with a desulfurization reaction and FLYASH discharged from an electric dust collector. In particular, in recent years, from the viewpoint of environmental consideration, it is necessary to consider economic factors that vary over time, such as environmental charge, and it is desired to grasp an optimum operation condition in consideration of such factors.

For example, Patent Document 1 discloses a technique for economically operating a desulfurization apparatus by considering, as a utility operation cost, a power cost factor based on the amount of limestone supplied to absorption liquid and the number of circulation pumps to be operated for circulating the absorption liquid, and also considering an income factor based on sales revenue of gypsum as a byproduct. Further, Patent Documents 2 and 3 disclose that the number of circulation pumps to be operated for circulating the absorption liquid is determined so as to minimize the operation cost while the $SO_2$ concentration on the outlet side of the desulfurization apparatus and the desulfurization ratio satisfy the required specifications.

Citation List

Patent Literature

     Patent Document 1: JP2011-110441A
     Patent Document 2: JP2000-015043A
     Patent Document 3: JP02-180615A

SUMMARY

Technical Problem

In Patent Documents 1 to 3, the evaluation of the operation condition is performed for the desulfurization apparatus in which the soundness is secured. However, in actual plant facilities, desulfurization performance may be reduced due to factors such as the effects of coexisting minor components due to fuel type changes (dissolution inhibition of limestone, oxidation inhibition, and the like) and facility blockage. If operation is continued in such a state, the symptoms become severe and the plant facility is forced to be stopped or the operation load is forced to be limited, and as a result, there is a risk of causing a great economic loss to the user.

It is possible to remotely monitor the operation state of a flue gas treatment facility including a desulfurization apparatus from a place (a management facility of a plant manufacturer or the like) geographically remote from the plant facility. However, in the desulfurization apparatus on site, by making an input amount of absorbent or a supply amount of absorption liquid excessively large at the discretion of the user, sufficient desulfurization performance is exhibited at a glance in terms of operation in the remote monitoring, and there is a concern that the discovery of the true performance deterioration may be delayed.

At least one embodiment of the present invention has been made in view of the above circumstances, and an object thereof is to provide an operation support system and an operation support method for a desulfurization apparatus capable of maximizing an economic benefit of a user due to operation of a plant facility while ensuring soundness of the desulfurization apparatus.

Solution to Problem

(1) To solve the abovementioned problem, an operation support system for a desulfurization apparatus according to at least one embodiment of the present invention is an operation support system for a desulfurization apparatus removing $SO_2$ from exhaust gas by using an absorbent, including an operation data input-acquisition unit configured to input and acquire operation data of the desulfurization apparatus (including input values of stationary analytic data, and hereinafter, called the operation data), an analytic performance calculation unit configured to calculate an ideal analytic performance concerning to a desulfurization performance of the desulfurization apparatus based on the operation data acquired by the operation data input-acquisition unit, a measured performance acquisition unit configured to acquire a measured performance when calculating the analytic performance (including not only that acquired automatically from a distributed control system (DCS) but also that manually input as a result of analysis, and hereinafter, called the measured performance), a soundness evaluation unit configured to evaluate soundness of the desulfurization apparatus by calculating a performance ratio of the measured performance acquired by the measured performance acquisition unit to the analytic performance calculated by the analytic performance calculation unit, and a support information creation unit configured to create support information including at least one operation condition candidate set based on an evaluation result of the soundness evaluation unit and a balance forecast corresponding to the operation condition candidate.

According to the configuration described above as (1), the analytic performance concerning to the desulfurization performance is obtained by calculation based on actual operation data of the desulfurization apparatus (which is a generic term for digital data measured by a large number of sensors incorporated in the apparatus). The analytic performance formula for performing the calculation is composed of component formulas related to chemical reactions such as residual concentration of the absorbent, an amount of $SO_2$ absorbed, a pH level, and liquid composition of coexisting components, and component formulas related to physical gas-liquid contact such as a ratio L/G of an amount of absorption liquid to an amount of exhaust gas, effective reaction height in an absorber, and an apparatus structure.

For example, the following (A) and (B) can be used as the analysis performance formula.

$$Y_{SO2out} = f\,(G,\,Y_{SO2in},\,[CaCO_3],\,L,\,k) \qquad (A)$$

$$\eta_{SO2} = g\,(G,\,Y_{SO2in},\,[CaCO_3],\,L,\,k) \qquad (B)$$

Here, $YSO_{2out}$ is a desulfurization apparatus outlet $SO_2$ concentration, $Y_{SO2in}$ is a desulfurization apparatus inlet $SO_2$ concentration, G is an amount of processed gas, $[CaCO_3]$ is a concentration of $CaCO_3$ in an absorber slurry, L is a slurry circulating flow rate, f and g are functions representing characteristics of the desulfurization apparatus, $\eta_{SO2}$ is a desulfurization ratio, and k is a reaction activity of the absorbent (dissolving rate). That is, the desulfurization apparatus outlet $SO_2$ concentration can be obtained by the function f with respect to the input value. This function f may be realized by a chemical reaction model as disclosed in Japanese Patent Application Laid-Open No. 59-199021 or Japanese Patent Application Laid-Open No. 63-229126, or it is also possible to measure characteristics of an actual desulfurization apparatus to be controlled under various operation conditions and to model the characteristics by a method such as statistical processing.

In the soundness evaluation unit, the soundness of the desulfurization apparatus is evaluated by performing comparison between the analytic performance and the measured performance corresponding thereto. In such evaluation of the soundness, the desulfurization performance can be absolutely evaluated regardless of the actual operation state on the user side, and the soundness can be accurately evaluated. The support information creation unit creates the support information including at least one operation condition set based on the evaluation result of the soundness evaluation unit and a balance forecast corresponding to the operation condition. By performing the operation based on the support information thus created, the user can perform the economical operation while ensuring the soundness of the desulfurization apparatus.

In the present specification, "analytic performance" broadly includes parameters corresponding to performances that can be calculated by inputting the operation data to an analytic performance formula (e.g., a simulation model).

(2) In some embodiments, in the configuration described above as (1), the support information creation unit sets a plurality of the operation condition candidates based on the evaluation result of the soundness evaluation unit and creates the support information by obtaining the balance forecast corresponding to each of the operation condition candidates.

According to the configuration described above as (2), the plurality of the operation condition candidates are set based on the soundness evaluation of the desulfurization apparatus and the balance forecast is obtained for each operation condition candidate. Thus, by comparing the balance forecasts of the plurality of the operation condition candidates, it is possible to provide operation support advantageous to the economic benefit of the user.

(3) In some embodiments, in the configuration described above as (1) or (2), the support information creation unit obtains the balance forecast in consideration of expense related to operation of the desulfurization apparatus and income from sale of byproducts generated during the operation of the desulfurization apparatus.

According to the configuration described above as (3), it is possible to provide operation support advantageous to the economic benefit of the user based on the balance forecast in consideration of expense related to operation of the desulfurization apparatus and income from sale of byproducts generated during the operation of the desulfurization apparatus.

(4) In some embodiments, in the configuration described above as (3), an operation condition to be adopted is selected from the plurality of operation condition candidates with priority given to minimizing the expense.

According to the configuration described above as (4), the economic benefit of the user can be optimized by selecting the operation condition so as to minimize expense factor that occupies a large proportion in the balance of the user.

(5) In some embodiments, in the configuration described above as any one of (1) to (4), the plurality of operation condition candidates are set so as to have different numbers of circulation pumps for circulating absorption liquid to the desulfurization apparatus.

According to the configuration described above as (5), by calculating, predicting, and comparing the apparent desulfurization performance and the operation cost in the case of changing to the state of the operation condition candidate in which the number of circulation pumps to be operated is different, it is possible to select the operation condition having the number of circulation pumps to be operated that effectively brings the economic benefit to the user while satisfying the performance. Since the circulation pumps occupy a relatively large proportion of the utility power cost, adopting of such an operation condition makes it possible to provide the operation support advantageous to the economic benefit of the user.

(6) In some embodiments, in the configuration described above as any one of (1) to (5), the soundness evaluation unit evaluates the soundness by calculating the performance ratio of the measured performance to the analytic performance and comparing the performance ratio with a predetermined reference value.

According to the configuration described above as (6), the soundness evaluation unit evaluates the soundness based on the performance ratio of the analytic performance and the measured performance corresponding thereto. With such a performance ratio, the desulfurization performance can be absolutely evaluated based on the measured performance of the desulfurization apparatus, and the soundness including that in a predictive stage can be accurately evaluated. Therefore, even when an input amount of absorbent or a supply amount of absorption liquid is excessively large at the discretion of the user, it is possible to accurately and promptly detect the soundness of the desulfurization apparatus based on the performance ratio.

(7) In some embodiments, in the configuration described above as any one of (1) to (6), the operation data is acquired from a local monitoring system via a network by a remote support system arranged at a position geographically remote from the local monitoring system capable of monitoring the desulfurization apparatus.

According to the configuration described above as (7), owing to that the remote support system, arranged at the position remote from the local monitoring system arranged at the site where the desulfurization apparatus is located, acquires the operation data used for soundness evaluation via a network, the operation support system can support the operation of the desulfurization apparatus in real time at the position remote from the site.

(8) In some embodiments, in the configuration described above as (7), the local monitoring system includes a display portion capable of displaying the support information acquired from the remote support system.

According to the configuration described above as (8), since the support information is displayed on the display portion included in the local monitoring system arranged at the user side, effective and rapid support for the user can be provided.

(9) To solve the abovementioned problem, an operation support method for a desulfurization apparatus according to at least one embodiment of the present invention is an operation support method for a desulfurization apparatus removing $SO_2$ from exhaust gas by using an absorbent, including steps of acquiring operation data of the desulfurization apparatus, calculating an analytic performance concerning to a desulfurization performance of the desulfurization apparatus based on the operation data, acquiring a measured performance when acquiring the analytic performance, evaluating soundness of the desulfurization apparatus by performing comparison between the analytic performance and the measured performance, setting at least one operation condition candidate based on the evaluation result of soundness, obtaining a balance forecast corresponding to the operation condition candidate, and creating support information including the operation condition candidate and the balance forecast.

The method described above as (9) can be appropriately performed by each operation support system (including the respective embodiments described above) for the desulfurization apparatus.

Advantageous Effects

According to at least one embodiment of the present invention, it is possible to provide an operation support system and an operation support method for a desulfurization apparatus capable of maximizing an economic benefit of a user due to operation of a plant facility while ensuring soundness of the desulfurization apparatus.

BRIEF DESCRIPTION OF DRAWINGS

FIG. 1 is a schematic diagram illustrating an overall configuration of an operation support system for a desulfurization apparatus according to an embodiment of the present invention.
FIG. 2 is a schematic diagram illustrating an example of a configuration layout of a local monitoring system, a network, and a remote support system of FIG. 1.
FIG. 3 is a block diagram functionally illustrating an internal configuration of a central server in the remote support system of FIG. 2.
FIG. 4 is a flowchart illustrating, for each step, an operation support method to be performed in the central server of FIG. 3.
FIG. 5 is a graph illustrating a change in a circulation amount of absorption liquid with the number of absorption liquid circulation pumps to be operated.
FIG. 6 illustrates an example of support information created in step S7 of FIG. 4.
FIG. 7 illustrates an example of auxiliary information included in the support information created in step S7 of FIG. 4.

DETAILED DESCRIPTION

Embodiments of the present invention will now be described in detail with reference to the accompanying drawings. It is intended, however, that unless particularly identified, dimensions, materials, shapes, relative positions and the like of components described in the embodiments shall be interpreted as illustrative only and not intended to limit the scope of the present invention.
For example, an expression of relative or absolute arrangement such as "in a direction", "along a direction", "parallel", "perpendicular", "centered", "concentric" and "coaxial" shall not be construed as indicating only the arrangement in a strict literal sense, but also includes a state where the arrangement is relatively displaced by a tolerance, or by an angle or a distance whereby it is possible to achieve the same function.
Further, for example, an expression of a shape such as a rectangular shape or a cylindrical shape shall not be construed as only the geometrically strict shape, but also includes a shape with unevenness or chamfered corners within the range in which the same effect can be achieved.
On the other hand, an expression such as "comprise", "include", "have", "contain" and "constitute" are not intended to be exclusive of other components.

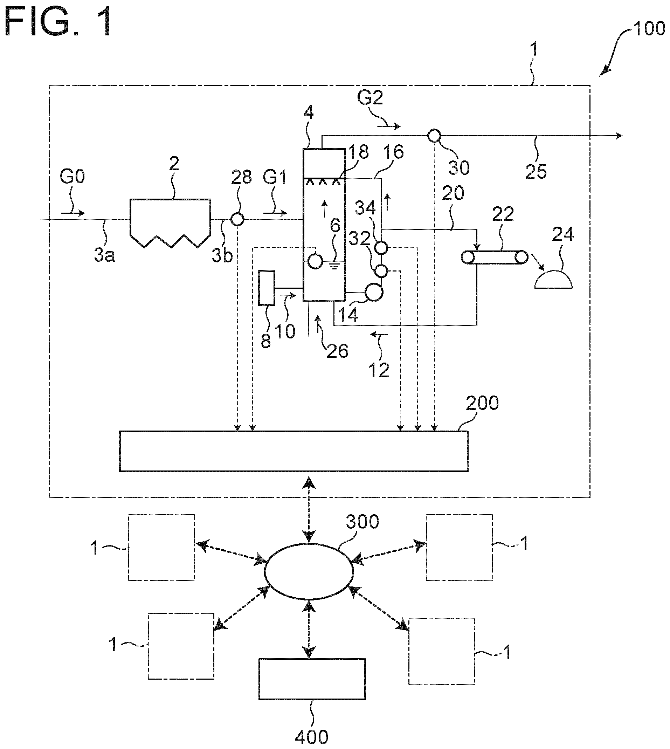

FIG. 1 is a schematic diagram illustrating an overall configuration of an operation support system 100 for a desulfurization apparatus 1 according to an embodiment of the present invention. In the operation support system 100, the desulfurization apparatus 1 included in a predetermined plant facility is set as a support target, and in the following embodiments, the case where remote support is performed at a point geographically remote from the plant facility is described as an example.
The desulfurization apparatus 1 includes a dust collecting device 2 installed in association with a boiler (not illustrated) of a plant facility such as a thermal power plant and for collecting fine particles contained in exhaust gas G0 flowing through an exhaust passage 3a of the boiler, and an absorption tower 4 installed downstream of the dust collecting device 2 in an exhaust passage 3b through which the exhaust gas G1 having passed through the dust collecting device 2 flows.
The dust collecting device 2 is an electric dust collector that charges fine particles contained in the exhaust gas G0 by performing corona discharging on the exhaust gas G0 supplied into a casing and collects dust by causing the fine particles to adhere to positively and negatively charged adhesion portions with electric attraction. The exhaust gas G1 subjected to a dust collecting process by the dust collecting device 2 is supplied to the absorption tower 4 through the exhaust passage 3b.

The absorption tower 4 performs a desulfurization process by absorbing $SO_2$ (sulfur dioxide) in the exhaust gas G1 by causing absorption liquid 6 containing limestone 10 to be in contact with the exhaust gas G1 subjected to the dust collecting process in the dust collecting device 2. The absorption liquid 6 is stored in the bottom of the absorption tower 4. The absorption liquid 6 is generated by mixing limestone 10 supplied from a limestone feeder 8 arranged outside the absorption tower 4 with water 12 supplied to the bottom of the absorption tower 4.

The absorption liquid 6 stored in the bottom of the absorption tower 4 is pressure-fed by an absorption liquid circulation pump 14 and supplied to an upper portion in the absorption tower 4 through an absorption liquid header 16 arranged outside the absorption tower 4. The absorption liquid circulation pump 14 includes a plurality of pump units connected in series or in parallel with each other, and is configured to be capable of variably adjusting the flow rate of the absorption liquid 6 pressure-fed from the absorption liquid circulation pump 14 by independently controlling an operation state of each pump unit. The absorption liquid 6 thus supplied to the upper portion of the absorption tower 4 contacts the exhaust gas G1 rising in the absorption tower 4 in a process of being sprayed and dropped from a nozzles 18 provided in the upper portion of the absorption tower 4. As a result, $SO_2$ contained in the exhaust gas G1 reacts with the limestone 10 in the absorption liquid 6, and the desulfurization process is performed.

The following formula (1) is a chemical reaction formula of the desulfurization process carried out in the absorption tower 4. In the desulfurization process, the limestone 10 and $SO_2$ contained in the exhaust gas G1 react with each other to generate gypsum 24 ($CaCO_4 \cdot 2H_2O$) as byproduct. The exhaust gas G2 from which $SO_2$ has been removed is discharged from the top of the absorption tower 4 to the outside through a desulfurization exhaust gas pipe 25.

$$SO_2 + 1/2O_2 + CaCO_3 + 2H_2O \rightarrow CaCO_4 \cdot 2H_2O + CO_2 \qquad (1)$$

In addition, a part of the absorption liquid 6 stored in the bottom of the absorption tower 4 is sent to a dehydrator 22 through an extraction pipe 20 branched from the absorption liquid header 16 outside the absorption tower 4 while being pressure-fed by the absorption liquid circulation pump 14. The dehydrator 22 is constituted by, for example, a belt filter, and the absorption liquid 6 is dehydrated in a process of being conveyed by the belt filter, and the generated gypsum 24 is discharged out of the system.

Note that the filtrate generated in the dehydration process in the dehydrator 22 is reused by being supplied as the water 12 to the bottom of the absorption tower 4.

Further, an oxidation air 26 is supplied to the bottom of the absorption tower 4. Thus, the oxidation air 26 is contained in the absorption liquid 6, so that oxidation of the sulfurous acid group generated as being transferred from the $SO_2$ exhaust gas into the absorption liquid 6 to the sulfuric acid group is promoted, and as a result, the removal efficiency of the $SO_2$ in the exhaust gas is also improved.

Incidentally, an inlet-side $SO_2$ concentration sensor 28 for detecting an $SO_2$ concentration of the exhaust gas G1 to be taken into the absorption tower 4, i.e., the inlet-side $SO_2$ concentration, is installed in the exhaust passage 3b connecting the dust collecting device 2 and the absorption tower 4. An outlet-side $SO_2$ concentration sensor 30 for detecting an $SO_2$ concentration of the exhaust gas G2 discharged from the absorption tower 4, i.e., the outlet-side $SO_2$ concentration, is installed in the desulfurization exhaust gas outlet pipe 25. In addition, a limestone concentration sensor 32 for detecting a limestone concentration of absorption liquid 6 in the absorption tower 4 and a pH sensor 34 for detecting a pH value thereof are installed in the absorption liquid header 16.

The detected values of these sensors are input to a control unit (not illustrated) of the desulfurization apparatus 1, and are used for operation control of the desulfurization apparatus 1. Specifically, operations of the limestone feeder 8 and the absorption liquid circulation pump 14, and supply of the oxidation air 26 are adjusted so that the detection values of the sensors each fall within a predetermined range, whereby the desulfurization apparatus 1 is controlled so that a desired desulfurization performance is exhibited.

The plant facility in which the desulfurization apparatus 1 having such a configuration is installed is provided with a local monitoring system 200 for monitoring the desulfurization apparatus 1. The local monitoring system 200 is located on the same site (within the site of the plant facility) as the desulfurization apparatus 1 to be monitored and is configured to be capable of communicating with a remote support system 400 being geographically remote via a network 300.

As illustrated in FIG. 1, the remote support system 400 is configured to be capable of communicating with a plurality of the local monitoring systems 200 respectively installed in a plurality of plant facilities via the network 300. Although the following description refers primarily to one specific local monitoring system 200, the same applies to other local monitoring systems 200 unless otherwise noted.

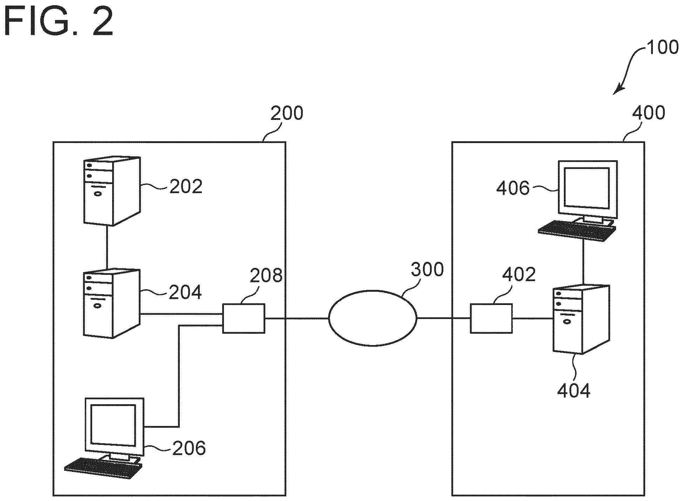

FIG. 2 is a schematic diagram illustrating an example of a configuration layout of the local monitoring system 200, the network 300, and the remote support system 400 of FIG. 1.

The local monitoring system 200 includes a monitoring server 202 for collecting operation data related to the desulfurization apparatus 1, a local server 204 for communicating various information handled in the local monitoring system 200 with the remote support system 400 via the network 300, and a client terminal 206 capable of accessing the local server 204.

In the monitoring server 202, the operation data related to the desulfurization apparatus 1 is collected by predetermined instruments at the installation site of the desulfurization apparatus 1. The operation data includes, for example, the detected values of the sensors illustrated in Fig. 1 (specifically, the inlet-side $SO_2$ concentration detected by the inlet-side $SO_2$ concentration sensor 28, the outlet-side $SO_2$ concentration detected by the outlet-side $SO_2$ concentration sensor 30, the limestone concentration of the absorption liquid 6 detected by the limestone concentration sensor 32, the pH value of the absorption liquid 6 detected by the pH sensor 34, and the like), and control signals to the limestone feeder 8, the absorption liquid circulation pump 14, and a supply device (not illustrated) of the oxidation air 26. The operation data collected by the monitoring server 202 is transmitted to the local server 204 via a local network.

The operation data received from the monitoring server 202 at the local server 204 is transmitted to the remote support system 400 via the network 300 as real-time data. The network 300 is connected, for example, through a dedicated line (Virtual Private Network: VPN), and the local server 204 is connected to the network 300 through a VPN router 208.

The local server 204 may store the operation data acquired from the monitoring server 202 as historical data for a predetermined period of time at a predetermined cycle. In this case, the local server 204 may process the historical data into data at a predetermined cycle and transmit the processed data to the remote support system 400 as real-time data.

The remote support system 400 is located geographically remote from the local monitoring system 200, for example, in a site of a plant manufacturer that is a manufacturer of plant equipment including the desulfurization apparatus 1, and is configured to communicate with the local monitoring system 200 by being connected to the network 300 through a VPN router 402. The remote support system 400 includes a central server 404 capable of communicating various information handled in the remote support system 400 with the local monitoring system 200 via the network 300 and a support terminal 406 capable of accessing the central server 404. The central server 404 and the support terminal 406 are configured to be capable of communicating with each other via a local network.

In the remote support system 400, remote assistance of the desulfurization apparatus 1 is provided based on the operation data received from the local monitoring system 200 via the network 300. The operation data received by the central server 404 may be stored in a data storage server (not illustrated) in the remote support system 400.

The operation data transmitted to the central server 404 can be accessed from the support terminal 406 as appropriate, and can be constantly checked by an operator for operating the support terminal 406 (e.g., plant manufacturer maintenance expert residing in the remote support system 400).

The operator performs analysis based on the operation data transmitted to the central server 404 using the support terminal 406, and transmits support information created based on the analysis results to a user of the plant facility (at the local monitoring system 200 side) as required.

In the local monitoring system 200, the support information is received from the remote support system 400 via the network 300. The support information received at the local monitoring system 200 can be appropriately confirmed by the user on the client terminal 206 through the local server 204.

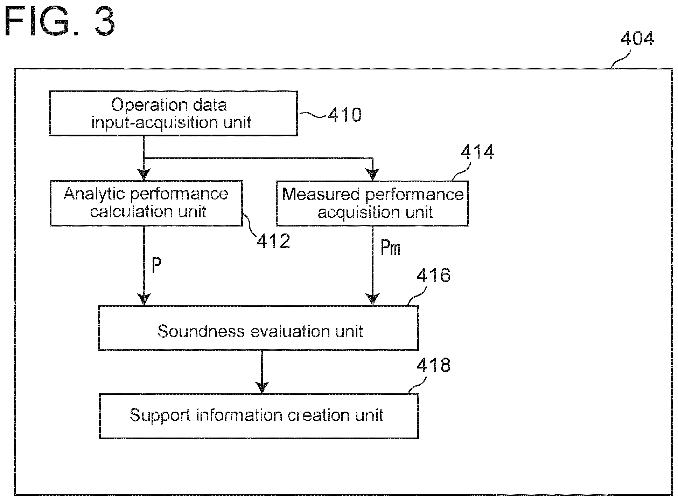

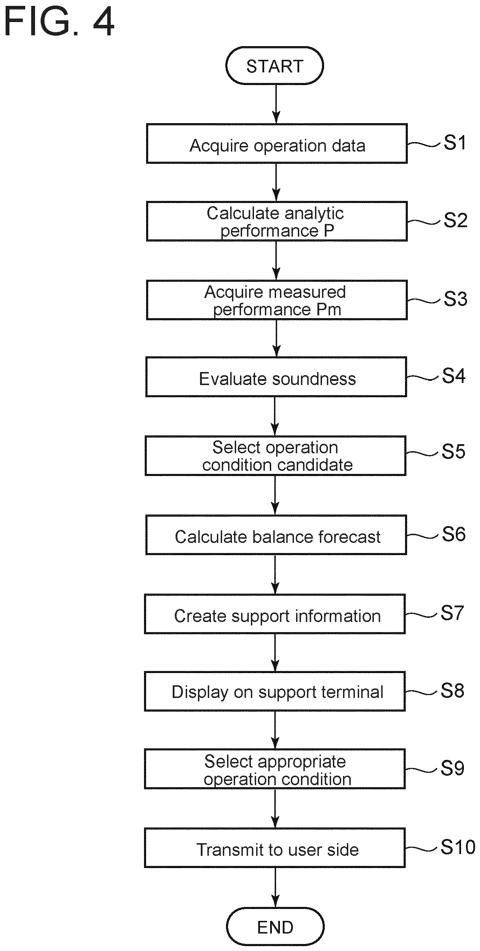

Subsequently, FIG. 3 is a block diagram functionally illustrating an internal configuration of the central server 404 in the remote support system 400 of FIG. 2, and FIG. 4 is a flowchart illustrating, for each step, an operation support method to be performed in the central server 404 of FIG. 3.

Here, at least a part of the internal configuration of the central server 404 illustrated in FIG. 3 may be distributed to the support terminal 406 constituting the remote support system 400 or may be constructed in a cloud server.

The central server 404 of the remote support system 400 includes an operation data input-acquisition unit 410 for acquiring the operation data of the desulfurization apparatus 1, an analytic performance calculation unit 412 for calculating an analytic performance P, a measured performance acquisition unit 414 for acquiring a measured performance Pm when acquiring the analytic performance P, a soundness evaluation unit 416 for evaluating soundness of the desulfurization apparatus 1 by calculating a performance ratio of the measured performance Pm to the analytic performance P, and a support information creation unit 418 for creating the support information based on an evaluation result of the soundness evaluation unit 416.

First, the operation data input-acquisition unit 410 acquires the operation data of the desulfurization apparatus 1 (step S1). As described above, the acquisition of the operation data by the operation data input-acquisition unit 410 is performed by receiving the real-time data transmitted from the local monitoring system 200 via the network 300.

The operation data acquired by the operation data input-acquisition unit 410 includes at least an amount of the absorbent. Here, the amount of the absorbent is, for example, a supply amount of the limestone 10 from the limestone feeder 8, and can be acquired by including the control signal to the limestone feeder 8 in the desulfurization apparatus 1 in the operation data. In this case, the supply amount of limestone 10 included in the operation data may be an amount necessary and sufficient to neutralize $SO_2$ (sulfur dioxide) in the exhaust gas G1 to be contacted with the absorption liquid 6, or may be an amount including a marginal amount added to the amount necessary and sufficient to sufficiently neutralize $SO_2$ (sulfur dioxide) in the exhaust gas G1.

The amount of the absorbent included in the operation data may be an amount of the absorbent (limestone 10) remaining in the absorption liquid 6. In this case, the remaining amount of the absorbent can be acquired as, for example, a limestone concentration of the absorption liquid 6 detected by the limestone concentration sensor 32.

As described above, the operation data acquired by the operation data input-acquisition unit 410 can broadly include the detected values of the sensors (specifically, the inlet-side $SO_2$ concentration detected by the inlet-side $SO_2$ concentration sensor 28, the outlet-side $SO_2$ concentration detected by the outlet-side $SO_2$ concentration sensor 30, the limestone concentration of the absorption liquid 6 detected by the limestone concentration sensor 32, and the pH value of the absorption liquid 6 detected by the pH sensor 34) and the control signals to the limestone feeder 8, the absorption liquid circulation pump 14, and the supply device (not illustrated) of the oxidation air 26.

Subsequently, the analytic performance calculation unit 412 calculates at least one analytic performance P based on the operation data acquired by the operation data input-acquisition unit 410 (step S2). The analytic performance P is calculated arithmetically as a parameter related to the desulfurization performance of the desulfurization apparatus 1. Here, an analytic performance formula is composed of constituent formulas related to chemical reactions such as residual concentration of the absorbent, an $SO_2$ absorbed amount, a pH value, liquid composition of coexisting components, based on the operation data, and constituent formulas related to physical gas-liquid contact such as a ratio L/G of an exhaust gas amount and a absorption liquid amount, effective reaction height in an absorber, an apparatus construction, and the like, and is set by comprehensively considering factors that may affect the desulfurization performance with respect to the amount of absorbent, for example.

The arithmetic formula to be used to determine the analytic performance P in step S2 is defined by a function having parameters included in the operation data acquired by the operation data input-acquisition unit 410 as variables, and allows parameter evaluation based on the actual operation data. Such a function is defined by evaluating correlations between the analytic performance P and each variable by a theoretical, experimental, or simulated method. Such an arithmetic formula may be stored in advance in a storage device (not illustrated) of the remote support system 400, or may be appropriately input by the operator of the remote support system 400.

The analytic performance P may be any parameter related to the desulfurization performance, and for example, a desulfurization ratio may be employed. Adopting the desulfurization ratio as the analytic performance P is preferable because soundness of the desulfurization apparatus 1 can be evaluated comprehensively, easily and accurately. Such an analytic performance may be calculated based on at least one of the structural parameter of the desulfurization apparatus 1 and the chemical reaction parameter of the absorbent (such as the limestone 10). This enables accurate estimation of the analytic performance concerning to the desulfurization performance from structural or chemical reactive aspects.

Subsequently, the measured performance acquisition unit 414 acquires the measured performance Pm corresponding to the analytic performance P calculated by the analytic performance calculation unit 412 (step S3). For example, when the analytic performance P corresponds to the desulfurization ratio as described above, the measured performance acquisition unit 414 acquires the measured performance Pm of the desulfurization ratio using the inlet-side $SO_2$ concentration detected by the inlet-side $SO_2$ concentration sensor 28 and the outlet-side $SO_2$ concentration detected by the outlet-side $SO_2$ concentration sensor 30.

Subsequently, the soundness evaluation unit 416 evaluates the soundness of the desulfurization apparatus 1 by comparing the analytic performance P calculated by the analytic performance calculation unit 412 with the measured performance Pm acquired by the measured performance acquisition unit 414 (step S4). Such soundness evaluation may be performed, for example, by calculating a performance ratio R, which is the ratio of the analytic performance P and the measured performance Pm, and determining whether or not performance degradation has occurred in the desulfurization apparatus 1 based on whether or not the performance ratio R is within an acceptable range defined by a predetermined reference value R0. The performance ratio R calculated in this manner is an index capable of absolutely evaluating the soundness of the desulfurization performance regardless of the operation state of the desulfurization apparatus 1 (for example, even when the apparent desulfurization ratio behaves so as to satisfy the threshold by setting the supply amount of the limestone 10 excessively on the user side).

In this case, the reference value R0 serving as an evaluation reference of the performance ratio R may be set according to specifications of the desulfurization apparatus 1 to be evaluated. As illustrated in FIG. 1, the remote support system 400 is connected to a plurality of different desulfurization apparatuses 1 via the network 300. Each of the desulfurization apparatuses 1 has inherent characteristics (habits) to no small extent. Such inherent characteristics may be reflected in the performance evaluation by selecting the reference value R0 corresponding to the desulfurization apparatus 1 to be evaluated from among the reference values corresponding to the apparatuses stored in the database in advance.

Here, the inherent characteristics (habits) of the desulfurization apparatus 1 may include not only initial characteristics but also a change in the performance evaluation result due to a change with time (ash stain, change in the type of coal, pump wear, etc.).

When the soundness of the desulfurization apparatus 1 is ideal, the analytic performance P and the measured performance Pm are equal to each other, and thus the performance ratio R becomes "1". On the other hand, when the soundness of the desulfurization apparatus 1 deviates from the ideal condition, a considerable difference occurs between the analytic performance P and the measured performance Pm, and thus the performance ratio R has a value deviating from "1". The reference value R0 is set as a threshold value for determining how much the performance ratio R deviates from the normal value "1" to determine that the soundness is impaired. For example, in the case where the reference value R0 is set to allow an error of up to -20% from the normal value "1" and when the performance ratio R is less than 0.8, the true performance based on the operation condition is apparently degraded and is determined to be in an unsound state.

Here, the performance ratio R used in the soundness evaluation may be a ratio between a logarithmic value of the analytic performance P and a logarithmic value of the measured performance Pm. In the desulfurization apparatus 1 having a high desulfurization performance (i.e., a desulfurization apparatus having a large $SO_2$ concentration differential between the inlet and the outlet), the use of a linear ratio as the performance ratio R results in a peaky property, and thus the use of a logarithmic ratio enables accurate diagnosis with high sensitivity.

Subsequently, at least one operation condition candidate is selected within the allowable range of the soundness of the desulfurization apparatus 1 (step S5). Here, the operation condition candidate means an operation condition that can be selected within a range in which the soundness of the desulfurization apparatus 1 evaluated in step S4 can be maintained in a favorable state. Such an operation condition candidate may include the current operation condition and may only be the current operation condition if there is no other possible operation condition. On the other hand, if there is another possible operation condition, the number of candidates may be arbitrary and may be singular or plural.

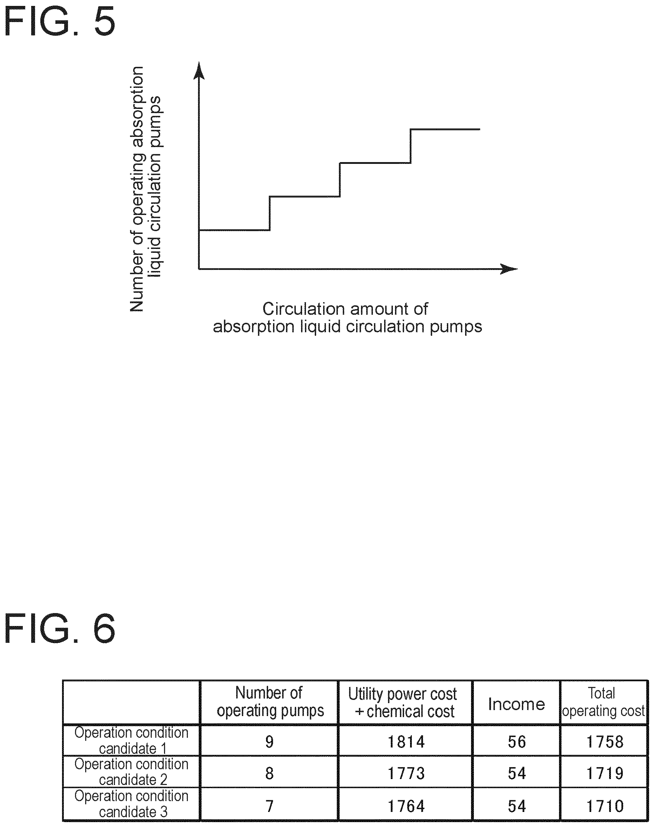

In the present embodiment described below, description will be provided on an example of operation condition candidates in which three operation condition candidates 1 to 3 each having different number of the absorption liquid circulation pumps 14 to be operated are selected, which significantly affect a utility power cost of the desulfurization apparatus 1. FIG. 5 is a graph illustrating a change in the circulation amount of the absorption liquid 6 with the number of the absorption liquid circulation pumps 14 to be operated. Since the change in the number of the absorption liquid circulation pumps 14 to be operated significantly affects the utility power cost of the desulfurization apparatus 1, it is possible to realize an economical operation that can minimize the utility power cost of the desulfurization apparatus 1 by selecting the operation condition candidate with a different number thereof to be operated.

Here, in the case where the absorption liquid circulation pump 14 is a variable capacity type, it is also possible to select the operation condition candidates each having a different capacity of the absorption liquid circulation pump 14. Further, in the case where the absorption liquid circulation pump 14 performs flow rate control of the absorption liquid 6 with rotation speed control by an inverter or movable blade control, the operation condition candidates in which the rotation speed control amount or the movable blade control amount is different may be selected.

Here, whether or not the soundness can be maintained in a favorable state in each operation condition candidate is determined, for example, by estimating how the analytic performance calculated in step S2 and the reference value set in step S3 change in each operation condition.

Further, the operation condition candidate may be selected on the condition that quality of the gypsum 24 generated as byproducts with the desulfurization reaction in the desulfurization apparatus 1 can be secured to a certain level or higher. The byproducts such as the gypsum 24 generated in the desulfurization apparatus 1 may be sold to a third party to serve as an income factor of a user. However, in order to sale them to a third party, a certain level or higher of quality desired by the third party is required. Therefore, the operation condition candidate may be added with a condition not only that the soundness of the desulfurization apparatus 1 can be maintained in a favorable state but also that the quality of byproducts can be sufficiently ensured. In particular, in selecting the operation condition candidate, it is also conceivable to maintain the soundness by increasing a calcium carbonate concentration in the absorption liquid 6. However, in this case, it should be noted that calcium carbonate in gypsum is increased due to excessive increase of the calcium carbonate concentration and gypsum purity is lowered, or that a gypsum moisture content is increased due to increase of fine particles, which may be lower than take-up quality of a gypsum trader.

Subsequently, a balance forecast is calculated for each operation condition candidate set in step S5 (step S6). The balance forecast is the balance predicted when the desulfurization apparatus 1 is operated according to each operation condition candidate, and is calculated as a total operating cost by considering expense related to the operation of the desulfurization apparatus 1 and income from sale of byproducts generated during the operation of the desulfurization apparatus 1.

The expense includes cost items required when operating the desulfurization apparatus 1 in accordance with each operation condition candidate, and includes, for example, a utility power cost, a chemical cost and an environmental charge. These cost items are calculated based on corresponding conversion rates. For example, the cost items such as the utility power cost, the chemical cost, and the environmental charge are calculated based on an electricity rate, a fuel and raw material rate, and an environmental charge rate, respectively.

On the other hand, the income includes income items according to sale of the byproducts generated during operation of the desulfurization apparatus 1, and includes, for example, sale profits of the gypsum 24 and FLYASH. The sale profits are calculated based on sale rates corresponding to sale objects. For example, sale profits for gypsum and FLYASH are calculated based on their sale rates respectively.

As the cost items and the rates used for calculating sale profits, information stored in a database in advance may be used, a value appropriately input by an operator may be used, or information acquired from the outside via, for example, a network may be used. Such rates are preferably updated at predetermined timing.

Subsequently, the support information creation unit 418 creates the support information including the operation condition candidate selected in step S5 and the balance forecast calculated in step S6 (step S7), and displays the support information on the support terminal 406 (step S8). Here, FIG. 6 illustrates an example of the support information created in step S7 of FIG. 4. In this example, the operation condition candidates 1 to 3 selected in step S5 are illustrated, and "utility power (expense)", "income (profit)", and "total operating cost (total balance)" are illustrated as the balance forecast corresponding to each operation condition candidate.

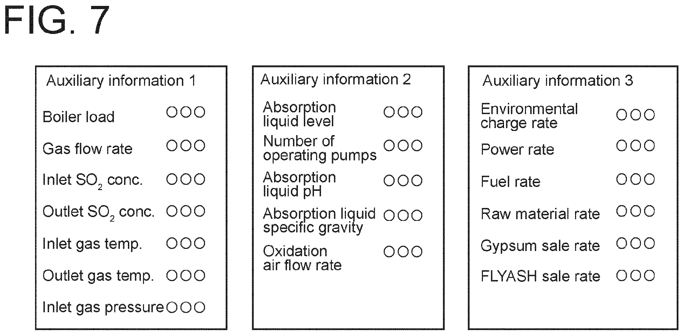

In addition to the balance forecast described above, the support information may also include auxiliary information for selecting an optimal operation condition from the operation condition candidates. FIG. 7 illustrates an example of the auxiliary information included in the support information created in step S7 of FIG. 4. In FIG. 7, the respective parameters indicating the state of the exhaust gas G1 to be subjected to the desulfurization process are illustrated as auxiliary information 1. Specifically, the instantaneous values of a boiler load located upstream the desulfurization apparatus 1, a flow rate of the exhaust gas G1, and the inlet $SO_2$ concentration, the outlet $SO_2$ concentration, inlet gas temperature, outlet gas temperature, and inlet gas pressure of desulfurization apparatus 1 are illustrated, respectively. Further, the parameters indicating the operation state of the desulfurization apparatus 1 are illustrated as auxiliary information 2. Specifically, the presently indicated values of a level of the absorption liquid 6 in the absorption tower 4, the number of operating absorption liquid circulation pumps 14, the pH value and the liquid specific gravity of the absorption liquid 6, the flow rate of the oxidation air 26 are illustrated, respectively. Further, rates serving as the basis for calculating the utility power cost and income are illustrated as auxiliary information 3. Specifically, an environmental charge rate, a power rate, a fuel rate, a raw material rate, and sale rates for gypsum and FLYASH are illustrated, respectively.

Such support information is displayed on the support terminal 406 of the remote support system 400 and is recognized by the operator of the support terminal 406. By referring to the support information displayed on the support terminal 406, the operator recognizes the current state of the desulfurization apparatus 1 and selects the appropriate operation condition for the user from the operation condition candidates (step S9).

A selection reference of the operation condition in step S9 is set by comprehensively considering the balance information and the parameters included in the support information. When emphasis is placed on keeping the total operating cost low, operation condition candidate 3 having the lowest total operating cost is selected in the example of Fig. 6. When emphasis is placed on keeping the utility power cost low, operation condition candidate 3 having the lowest utility power cost is selected. When emphasis is placed on securing a large amount of income, operation condition candidate 1 having the largest income is selected.

In a system such as a power generation plant where the main business is selling electricity to the outside, maximizing the amount of electricity selling is prioritized. In addition, a selling price of the gypsum 24 being byproducts of the desulfurization apparatus 1 is lower than the electricity selling price. In view of these circumstances, it is possible to select an operation condition that maximizes the user's business benefit by selecting the operation condition candidate that minimizes the utility power cost (expense).

In the selection of the operation condition candidate, by considering the auxiliary information included in the support information, an operation condition suitable for the user may be selected in consideration of various factors not limited to the balance information. This makes it possible to make comprehensive determination considering not only the simple balance but also the operation state of the boiler and the desulfurization apparatus 1 in the background or external factors such as the environmental charge rate and sale rate of byproducts.

In particular, since sale of the gypsum 24, which is an element of the income, is often performed batchwise, it may be considered that there is some gap between the current sale rate and the actual rate at sale.

Further, the selection reference of the operation condition in step S9 may be set to be variable in accordance with the surrounding environment. For example, a selection reference for the operation condition prioritizing the soundness may be adopted when the environmental charge rate is high, and a cost-oriented selection reference prioritizing the total operating cost over the soundness may be adopted when the environmental charge rate is low. This enables the selection of an operation condition that provides an economic benefit in total while ensuring the soundness of the desulfurization apparatus 1 in accordance with changes in the surrounding environment. This is effective in improving user's profitability.

Here, the selection of the operation condition in step S9 may be performed by an artificial determination of the operator of the support terminal 406, or may be performed automatically by the central server 404 or the support terminal

EP 3 747 533 A1

406.

Subsequently, the operation condition selected by the support terminal 406 is transmitted to the local monitoring system 200 via the network 300 and displayed on the client terminal 206 (step S10). Thus, the operation condition selected by the operator of the remote support system 400 is displayed on the client terminal 206. The user recognizes the operation condition displayed on the client terminal 206 and executes operation in accordance with the operation condition, thereby enabling economical operation of the desulfurization apparatus 1.

In the present embodiment, description is provided on the case where the operator selects the operation condition while the support information created in step S7 is displayed on the support terminal 406 side of the remote support system 400. However, the support information created in step S7 may be directly displayed on the client terminal 206 to give the user side a right to select operation condition.

As described above, according to the above-described embodiment, the soundness of the desulfurization apparatus 1 is monitored at the remote support system 400 and the appropriate operation condition is selected for the user, so that the plant operation on the user side can be remotely supported. Such selection of the operation condition is performed in accordance with needs of the user within a range in which the soundness of the desulfurization apparatus 1 can be secured. Even when the fuel type of the desulfurization apparatus 1 is changed or the like, it is possible to effectively support the economic operation that maximizes the profitability of the user in consideration of the surrounding environment.

Industrial Applicability

At least one embodiment of the present invention is available for an operation support system and an operation support method for a desulfurization apparatus using absorbent to remove $SO_2$ in exhaust gas.

Reference Signs List

| | |
|---|---|
| 1 | Desulfurization apparatus |
| 2 | Dust collecting device |
| 3a, 3b | Exhaust passage |
| 4 | Absorption tower |
| 6 | Absorption liquid |
| 8 | Limestone feeder |
| 10 | Limestone |
| 12 | Water |
| 14 | Absorption liquid circulation pump |
| 16 | Absorption liquid header |
| 18 | Nozzle |
| 20 | Extraction pipe |
| 22 | Dehydrator |
| 24 | Gypsum |
| 25 | Desulfurization exhaust gas pipe |
| 26 | Oxidation air |
| 100 | Operation support system |
| 200 | Local monitoring system |
| 202 | Monitoring server |
| 204 | Local server |
| 206 | Client terminal |
| 300 | Network |
| 400 | Remote support system |
| 404 | Central server |
| 406 | Support terminal |
| 410 | Operation data input-acquisition unit |
| 412 | Analytic performance calculation unit |
| 414 | Measured performance acquisition unit |
| 416 | Soundness evaluation unit |
| 418 | Support information creation unit |

**Claims**

1. An operation support system for a desulfurization apparatus removing $SO_2$ from exhaust gas by using an absorbent, comprising:

   an operation data input-acquisition unit configured to acquire operation data of the desulfurization apparatus;
   an analytic performance calculation unit configured to calculate an ideal analytic performance concerning to a desulfurization performance of the desulfurization apparatus based on the operation data acquired by the operation data input-acquisition unit;
   a measured performance acquisition unit configured to acquire a measured performance when acquiring the analytic performance;
   a soundness evaluation unit configured to evaluate soundness of the desulfurization apparatus by calculating a performance ratio of the measured performance acquired by the measured performance acquisition unit to the analytic performance calculated by the analytic performance calculation unit; and
   a support information creation unit configured to create support information including at least one operation condition candidate set based on an evaluation result of the soundness evaluation unit and a balance forecast corresponding to the operation condition candidate.

2. The operation support system for a desulfurization apparatus according to claim 1, wherein the support information creation unit sets a plurality of the operation condition candidates based on the evaluation result of the soundness evaluation unit and creates the support information by obtaining the balance forecast corresponding to each of the operation condition candidates.

3. The operation support system for a desulfurization apparatus according to claim 1 or 2, wherein the support information creation unit obtains the balance forecast in consideration of expense related to operation of the desulfurization apparatus and income from sale of byproducts generated during the operation of the desulfurization apparatus.

4. The operation support system for a desulfurization apparatus according to claim 3, wherein an operation condition to be adopted is selected from the plurality of operation condition candidates with priority given to minimizing the expense.

5. The operation support system for a desulfurization apparatus according to any one of claims 1 to 4, wherein the plurality of operation condition candidates are set so as to have different numbers of circulation pumps to be operated for circulating absorption liquid to the desulfurization apparatus.

6. The operation support system for a desulfurization apparatus according to any one of claims 1 to 5, wherein the soundness evaluation unit evaluates the soundness by calculating the performance ratio of the measured performance to the analytic performance and comparing the performance ratio with a predetermined reference value.

7. The operation support system for a desulfurization apparatus according to any one of claims 1 to 6, wherein the operation data is acquired from a local monitoring system via a network by a remote support system arranged at a position geographically remote from the local monitoring system capable of monitoring the desulfurization apparatus.

8. The operation support system for a desulfurization apparatus according to claim 7, wherein the local monitoring system includes a display portion capable of displaying the support information acquired from the remote support system via the network.

9. An operation support method for a desulfurization apparatus removing $SO_2$ from exhaust gas by using an absorbent, comprising steps of:

   acquiring operation data of the desulfurization apparatus;
   calculating an analytic performance concerning to a desulfurization performance of the desulfurization apparatus based on the operation data;
   acquiring a measured performance when acquiring the analytic performance;
   evaluating soundness of the desulfurization apparatus by performing comparison between the analytic performance and the measured performance;
   setting at least one operation condition candidate based on an evaluation result of the soundness;
   obtaining a balance forecast corresponding to the operation condition candidate; and

creating support information including the operation condition candidate and the balance forecast.

# FIG. 1

FIG. 2

# FIG. 3

404

Operation data input-acquisition unit — 410

Analytic performance calculation unit — 412

Measured performance acquisition unit — 414

P

Pm

Soundness evaluation unit — 416

Support information creation unit — 418

# FIG. 4

START

↓

Acquire operation data — S1

↓

Calculate analytic
performance P — S2

↓

Acquire measured
performance Pm — S3

↓

Evaluate soundness — S4

↓

Select operation
condition candidate — S5

↓

Calculate balance forecast — S6

↓

Create support information — S7

↓

Display on support terminal — S8

↓

Select appropriate
operation condition — S9

↓

Transmit to user side — S10

↓

END

# FIG. 5

Number of operating absorption
liquid circulation pumps

Circulation amount of
absorption liquid circulation pumps

# FIG. 6

|  | Number of operating pumps | Utility power cost + chemical cost | Income | Total operating cost |
|---|---|---|---|---|
| Operation condition candidate 1 | 9 | 1814 | 56 | 1758 |
| Operation condition candidate 2 | 8 | 1773 | 54 | 1719 |
| Operation condition candidate 3 | 7 | 1764 | 54 | 1710 |

# FIG. 7

| Auxiliary information 1 | |
|---|---|
| Boiler load | ○○○ |
| Gas flow rate | ○○○ |
| Inlet $SO_2$ conc. | ○○○ |
| Outlet $SO_2$ conc. | ○○○ |
| Inlet gas temp. | ○○○ |
| Outlet gas temp. | ○○○ |
| Inlet gas pressure | ○○○ |

| Auxiliary information 2 | |
|---|---|
| Absorption liquid level | ○○○ |
| Number of operating pumps | ○○○ |
| Absorption liquid pH | ○○○ |
| Absorption liquid specific gravity | ○○○ |
| Oxidation air flow rate | ○○○ |

| Auxiliary information 3 | |
|---|---|
| Environmental charge rate | ○○○ |
| Power rate | ○○○ |
| Fuel rate | ○○○ |
| Raw material rate | ○○○ |
| Gypsum sale rate | ○○○ |
| FLYASH sale rate | ○○○ |

## INTERNATIONAL SEARCH REPORT

| | International application No. |
|---|---|
| | PCT/JP2019/007893 |

| A. CLASSIFICATION OF SUBJECT MATTER |
|---|
| Int.Cl. B01D53/50(2006.01)i, B01D53/14(2006.01)i, B01D53/78(2006.01)i |
| According to International Patent Classification (IPC) or to both national classification and IPC |

| B. FIELDS SEARCHED |
|---|
| Minimum documentation searched (classification system followed by classification symbols) |
| Int.Cl. B01D53/34-53/85, B01D53/14-53/18, G06Q50/00-50/20, G06Q50/26-99/00 |

| Documentation searched other than minimum documentation to the extent that such documents are included in the fields searched | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

| Electronic data base consulted during the international search (name of data base and, where practicable, search terms used) |
|---|
| |

| C. DOCUMENTS CONSIDERED TO BE RELEVANT | | |
|---|---|---|
| Category* | Citation of document, with indication, where appropriate, of the relevant passages | Relevant to claim No. |
| Y | JP 2011-110440 A (MITSUBISHI HEAVY INDUSTRIES, LTD.) 09 June 2011, claims, paragraphs [0031]-[0070], fig. 1-5 & WO 2011/065118 A1 | 1-9 |
| Y | JP 2011-110441 A (MITSUBISHI HEAVY INDUSTRIES, LTD.) 09 June 2011, claims, paragraphs [0017]-[0070], fig. 1-5 & WO 2011/065119 A1 | 1-9 |
| A | US 2006/0047526 A1 (ALSTOM TECHNOLOGY LTD.) 02 March 2006 (Family: none) | 1-9 |
| A | JP 10-85549 A (BABCOCK HITACHI KK) 07 April 1998 (Family: none) | 1-9 |

☐ Further documents are listed in the continuation of Box C.    ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 09 May 2019 (09.05.2019) | 21 May 2019 (21.05.2019) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011110441 A
- JP 2000015043 A
- JP 2180615 A
- JP 59199021 A
- JP 63229126 A